Europäisches Patentamt

European Patent Office

Office européen des brevets

(11) Veröffentlichungsnummer: **0 174 626**
**A2**

(12) **EUROPÄISCHE PATENTANMELDUNG**

(21) Anmeldenummer: **85111343.1**

(22) Anmeldetag: **07.09.85**

(51) Int. Cl.⁴: **C 12 N 1/02**
**C 12 P 19/06**

(30) Priorität: **13.09.84 DE 3433611**

(43) Veröffentlichungstag der Anmeldung:
**19.03.86 Patentblatt 86/12**

(84) Benannte Vertragsstaaten:
**AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Goertz, Hans-Helmut, Dr.**
**Am Wurmberg 11**
**D-6713 Freinsheim(DE)**

(72) Erfinder: **Lungershausen, Rolf, Dr.**
**Prager Strasse 29**
**D-6700 Ludwigshafen(DE)**

(72) Erfinder: **Marcinowski, Stefan, Dr.**
**Weimarer Strasse 82**
**D-6700 Ludwigshafen(DE)**

(72) Erfinder: **Sanner, Axel, Dr.**
**Lorscher Ring 2c**
**D-6710 Frankenthal(DE)**

(54) **Verfahren zur Abtrennung von Zellmasse.**

(57) Es wird ein Verfahren zur Abtrennung von Zellmasse aus wäßrigen Systemen beschrieben, welches darin besteht, daß man das wäßrige, die Zellmasse enthaltende System mit einer in Wasser nicht oder nur wenig löslichen organischen Flüssigkeit vermischt und nach Phasentrennung die wäßrige Phase von der organischen Phase und der als Zwischenphase vorliegenden Zellmasse abtrennt.

EP 0 174 626 A2

Verfahren zur Abtrennung von Zellmasse

Die Erfindung betrifft ein Verfahren zur Abtrennung von Zellmasse aus wäßrigen Systemen.

Bei der Aufarbeitung von Fermentationslösungen ist es in vielen Fällen notwendig, die Zellmasse abzutrennen. Hierfür sind verschiedene Methoden bekannt, so beispielsweise Zentrifugation, Filtration mit oder ohne vorherige Flockung oder Lyse mit Hilfe bestimmter Enzyme. Besonders schwierig ist die Abtrennung der Zellmasse aus Brühen, die - beispielsweise infolge einer darin enthaltenen hochmolekularen Substanz - eine hohe Viskosität aufweisen. In diesen Fällen wird die Abtrennung der Zellmasse durch Zentrifugation durch die hohe Viskosität außerordentlich erschwert, wenn nicht unmöglich. Es muß dann bei so hohen g-Werten zentrifugiert werden, daß eine Übertragung in den technischen Maßstab nicht realisierbar oder unwirtschaftlich ist, oder die Lösung muß stark verdünnt werden, was im allgemeinen sehr unerwünscht ist. Eine Abtrennung durch Filtration erfordert den Einsatz von Membranfiltern mit auf die Zellgröße abgestimmten Porenweiten im Bereich von ca. 0,2 bis 10 µm. Die hohen Viskositäten führen hier zu geringen Filtrationsgeschwindigkeiten, wobei im Laufe der Filtration eine auf dem Filter sich bildende Gelschicht aus Zellmasse die Filtration völlig zum Erliegen bringen kann.

Die Verwendung hochmolekularer Flockungsmittel, wie sie aus der Abwasserklärung bekannt ist, ist nicht zur Lösung des Problems geeignet, weil die in der Fermentationslösung enthaltene polymere Substanz nicht durch ein zweites Polymeres verunreinigt werden soll.

Der vorliegenden Erfindung lag die Aufgabe zugrunde, ein möglichst einfaches Verfahren zur Abtrennung von Zellmasse aus Lösungen zu entwickeln, das die Nachteile der oben genannten Verfahren vermeidet.

Gegenstand der Erfindung ist ein Verfahren zur Abtrennung von Zellmasse aus wäßrigen Systemen, welches darin besteht, daß man das wäßrige, die Zellmasse enthaltende System mit einer in Wasser nicht oder nur wenig löslichen organischen Flüssigkeit vermischt und nach Phasentrennung die wäßrige Phase von der organischen Phase und der als Zwischenphase vorliegenden Zellmasse abtrennt.

Zellmasse ist hier als übergeordneter Begriff für Zellen von Mikroorganismen wie Bakterien, Hefen oder Pilze sowie Zellen pflanzlicher oder tierischer Herkunft zu verstehen. Es kann sich um lebende oder tote Zellen oder um Zellfragmente handeln.

WK/P

Die wäßrigen Systeme, aus denen die Zellmasse abgetrennt werden soll, sind vorzugsweise Lösungen, wie sie bei Fermentationen anfallen, es können jedoch auch sonstige Zellen enthaltende Lösungen sein.

Ein Zellmasse enthaltendes wäßriges System der genannten Art wird erfindungsgemäß mit einer wasserunlöslichen organischen Flüssigkeit vermischt. Die Vermischung kann hierbei durch intensives Schütteln oder Rühren erfolgen, beispielsweise mit Hilfe eines Schnellrührers, eines Ultra-Turrax[(R)] oder eines Mixers. Die Durchmischung kann absatzweise erfolgen, oder auch kontinuierlich, indem beispielsweise das wäßrige System und die organische Flüssigkeit durch eine Kolloidmühle geleitet werden. Es kann zweckmäßig sein, die Durchmischung bei erhöhter Temperatur vorzunehmen.

Unter den erfindungsgemäß zu verwendenden in Wasser nicht oder nur wenig löslichen organischen Flüssigkeiten sind solche organischen Flüssigkeiten zu verstehen, die mit Wasser nicht unbegrenzt mischbar sind und sich vorzugsweise zu nicht mehr als 10 % in Wasser lösen. Die organische Flüssigkeit kann eine höhere oder eine niedrigere Dichte als die wäßrige Phase besitzen  Ein deutlicher Dichtenunterschied ist im allgemeinen für die spätere Phasentrennung von Vorteil. Unter den Flüssigkeiten mit höherer Dichte als Wasser sind vor allem Chlorkohlenwasserstoffe zu nennen, insbesondere 1,1,1-Trichlorethan und Methylenchlorid, unter denen mit niedrigerer Dichte Kohlenwasserstoffe wie Cyclohexan oder Toluol. Aber auch organische Flüssigkeiten aus anderen Substanzklassen kommen in Betracht, beispielsweise Ether wie Diethylketon oder Cyclohexanon, Ester wie Butyl- oder Ethylacetat, höhere Alkohole wie n-Butanol. Auch Mischungen solcher Flüssigkeiten kommen in Frage. Welche Flüssigkeit am besten geeignet ist, hängt vom Einzelfall ab und läßt sich in einem einfachen Versuch klären.

Die eingesetzte Menge der organischen Flüssigkeit hängt vom Einzelfall ab. Sie beträgt vorzugsweise zwischen 10 und 100 Vol.% der wäßrigen Phase, aber auch andere Volumenverhältnisse sind möglich. Es kann im Einzelfall zweckmäßig sein, der organischen Flüssigkeit oder der wäßrigen Phase eine oberflächenaktive Substanz zuzusetzen. Auch die Mitverwendung wasserlöslicher organischer Flüssigkeiten kann vorteilhaft sein, wenn sie in solchen Mengenverhältnissen erfolgt, daß eine Phasentrennung organisch-wäßrig gewährleistet bleibt. Im Einzelfall kann auch das Auflösen von Salzen in der wäßrigen Phase von Vorteil sein.

Nach dem Vermischen liegt das System als mehr oder weniger stabile Emulsion vor, die sich im allgemeinen nur sehr langsam in seine Phasen trennt. Durch Zentrifugation läßt sich die Phasentrennung jedoch sehr leicht bewerkstelligen. Überraschenderweise tritt bei Verwendung einer geeigneten organischen Flüssigkeit nicht nur eine Trennung der beiden
flüssigen Phasen ein, sondern es bildet sich gleichzeitig eine Art
Zwischenphase aus, in der sich der größte Teil der Zellmasse befindet.
Die über- oder unterstehende wäßrige Phase weist nur noch in wesentlich
geringerem Maße die für zellhaltige Lösungen kennzeichnende Trübung auf.

Die Abtrennung der wäßrigen Phase kann durch Dekantieren erfolgen. Es ist
aber auch möglich, Phasentrennung und Abtrennung der wäßrigen Phase in
einem Schritt in einem Dekanter durchzuführen.

Das neue Verfahren führt zu einer schnelleren Abtrennung der Zellmasse als
eine Zentrifugation ohne Zugabe von organischer Flüssigkeit. Es eignet
sich vorzugsweise für Systeme, deren Viskosität über etwa 500 mPas beträgt. Besonders überraschend ist die Tatsache, daß mit dessen Hilfe auch
die Abtrennung von Zellmasse aus hochviskosen Fermentationslösungen möglich ist, wie sie beispielsweise bei der fermentativen Herstellung von
hochmolekularen Polysacchariden anfallen. Solche Lösungen haben Viskositäten von mehreren Pas oder darüber. Dennoch gelingt die Abtrennung der
Zellmasse nach dem neuen Verfahren. Werden eine unbehandelte Fermentationslösung dieser Art und eine erfindungsgemäß mit organischer Flüssigkeit behandelte unter gleichen Bedingungen zentrifugiert, so ist bei der
unbehandelten Probe kein Absetzen der Zellmasse zu beobachten, während
sie sich bei der behandelten Probe in der "Zwischenphase" ansammelt.

Die folgenden Beispiele dienen der Veranschaulichung der Erfindung.

Beispiel 1

Eine Fermentationslösung von Bacillus subtilis wurde mit gleichen Teilen
Methylenchlorid intensiv gerührt und anschließend in einer Laborzentrifuge kurz zentrifugiert. Die organische und die wäßrige Phase hatten sich
getrennt. Zwischen beiden hatte sich die Zellmasse als pastöse Zwischenschicht abgesetzt.

Beispiel 2

Zu 100 g einer Fermentationslösung von Xanthomonas campestris mit einer
Viskosität von 10 Pas wurden 50 ml Methylenchlorid gegeben. Die Mischung
wurde 1 min mit einem Ultra-Turrax[(R)] behandelt, anschließend wurde die
milchige Mischung 20 min in einer Laborzentrifuge bei 5000 rpm zentrifugiert. Hiernach hatte sich das Methylenchlorid klar abgesetzt, die überstehende viskose wäßrige Lösung war deutlich weniger durch Bakterien getrübt als vor der Behandlung, zwischen den Phasen hatte sich eine verfestigte Schicht von Zellmasse abgesetzt, von der die wäßrige Phase abgehebert werden konnte.

Beispiel 3

Es wurde wie in Beispiel 2 verfahren, jedoch statt Methylenchlorid 1,1,1-Trichlorethan verwendet. Das Trennergebnis war das gleiche.

Beispiel 4

Es wurde wie in Beispiel 2 verfahren, jedoch statt Methylenchlorid Toluol verwendet. Nach der Zentrifugation war die obere (organische) Phase milchig und ging in die Zwischenzone der verfestigten Zellmasse über.

Beispiel 5

Es wurde wie in Beispiel 4 verfahren, jedoch statt Toluol Cyclohexan verwendet. Das Trennergebnis war das gleiche.

Beispiel 6

Zu 100 g einer Fermentationslösung von Sclerotium rucanicum mit einer Viskosität von 5 Pas wurden 20 ml Methylenchlorid gegeben und die Mischung 1 min mit einem Ultra-Turrax$^{(R)}$ behandelt Anschließend wurde 20 min in einer Laborzentrifuge bei 500 rpm zentrifugiert. Hiernach hatte sich das Methylenchlorid klar abgesetzt, die überstehende viskose wäßrige Lösung war deutlich klarer.

Zwischen den Phasen hatte sich eine kompakte Schicht von Zellmasse abgesetzt, von der sich die wäßrige Phase abdekantieren ließ.

Beispiel 7

Es wurde wie in Beispiel 6 verfahren, jedoch statt Methylenchlorid 1,1,1-Trichlorethan verwendet. Das Trennergebnis war das gleiche.

Patentansprüche

1.- Verfahren zur Abtrennung von Zellmasse aus wäßrigen Systemen, dadurch gekennzeichnet, daß man das wäßrige, die Zellmasse enthaltende System mit einer in Wasser nicht oder nur wenig löslichen organischen Flüssigkeit vermischt und nach Phasentrennung die wäßrige Phase von der organischen Phase und der als Zwischenphase vorliegenden Zellmasse abtrennt.

2. Verfahren nach Anspruch 1, dadurch gekennzeichnet, daß die wäßrige Phase eine Fermentationslösung eines Bakteriums oder eines Pilzes ist.

3. Verfahren nach Anspruch 1, dadurch gekennzeichnet, daß die Viskosität des wäßrigen Systems größer als 500 mPas ist.

4. Verfahren nach Anspruch 1, dadurch gekennzeichnet, daß die organische Flüssigkeit ein Chlorkohlenwasserstoff ist.

5. Verfahren nach Anspruch 1, dadurch gekennzeichnet, daß die organische Flüssigkeit Methylenchlorid oder 1,1,1-Trichlorethan ist.

6. Verfahren nach Anspruch 1, dadurch gekennzeichnet, daß die organische Flüssigkeit ein Kohlenwasserstoff ist.

7. Verfahren nach Anspruch 1, dadurch gekennzeichnet, daß die organische Flüssigkeit Cyclohexan oder Toluol ist.